# EUROPÄISCHE PATENTSCHRIFT

(11) **EP 2 175 975 B1**
(45) Veröffentlichungstag und Bekanntmachung des Hinweises auf die Patenterteilung: **20.06.2012**
(21) Anmeldenummer: 08774531.1
(22) Anmeldetag: 30.06.2008
(51) Int. Cl.: B01J 2/30, C07C 227/44, C11D 3/33, C11D 3/37, B01J 2/22, C11D 17/00, C11D 7/32

(54) **VERFAHREN ZUR HERSTELLUNG EINES RIESELFÄHIGEN UND LAGERSTABILEN FESTSTOFFES ENTHALTEND IM WESENTLICHEN ALPHA-ALANIN-N,N-DIESSIGSÄURE UND/ODER EIN ODER MEHRERE DERIVATE DER ALPHA-ALANIN-N,N-DIESSIGSÄURE**
PROCESS FOR PRODUCING A FREE-FLOWING AND STORAGE-STABLE SOLID COMPRISING ESSENTIALLY ALPHA-ALANINE-N,N-DIACETIC ACID AND/OR ONE OR MORE DERIVATIVES OF ALPHA-ALANINE-N,N-DIACETIC ACID
PROCÉDÉ DE FABRICATION D'UN SOLIDE VERSABLE ET STABLE À L'ENTREPOSAGE, CONTENANT ESSENTIELLEMENT DE L'ACIDE LPHA-ALANINE-N,N-DIACÉTIQUE ET/OU UN OU PLUSIEURS DÉRIVÉS DE L'ACIDE LPHA-ALANINE-N,N-DIACÉTIQUE

(30) Priorität: 03.07.2007 EP 07111575
(43) Veröffentlichungstag der Anmeldung: 21.04.2010
(73) Patentinhaber: BASF SE, 67056 Ludwigshafen (DE)
(72) Erfinder: HEINZ, Robert, 67067 Ludwigshafen (DE); FLORE, Karin, 67549 Worms (DE); KISSAU, Lars, 67157 Wachenheim (DE); HEIDENFELDER, Thomas, 67125 Dannstadt-Schauernheim (DE); SEEBECK, Tanja, 64625 Bensheim (DE); MRZENA, Frank, 67112 Mutterstadt (DE)
(86) Internationale Anmeldenummer: PCT/EP2008/058377
(87) Internationale Veröffentlichungsnummer: WO 2009/003979

(56) Entgegenhaltungen:
- WO-A-2006/002954
- DE-A1- 19 937 345

## Beschreibung

Die Erfindung betrifft ein Verfahren zur Herstellung eines rieselfähigen und lagerstabilen Feststoffes enthaltend im Wesentlichen α-Alanin-N,N-diessigsäure und/oder ein oder mehrere Derivate der α-Alanin-N,N-diessigsäure sowie eine Verwendung des nach dem Verfahren hergestellten Feststoffes.

α-Alanin-N,N-diessigsäure und ihre Derivate sind als biologisch abbaubare Komplexbildner für Erdalkali und Schwermetallionen in Reinigungsformulierungen der Geschirrreinigung unter dem Markennamen Trilon^{®} M der BASF AG bekannt und beispielsweise in EP-A 0 781 762 beschrieben.

α-Alanin-N,N-diessigsäure wird durch Sprühtrocknung eines entsprechenden Reaktionsgemisches hergestellt und dabei als amorphes, extrem hygroskopisches Pulver erhalten, das als solches auf Grund seiner Hygroskopizität nicht direkt für den Einsatz zur Herstellung von Tabs für Spülmaschinen durch Vermischen mit weiteren Komponenten, insbesondere Enzymen, Peroxyden, Soda, usw. und Verpressen der Mischung zu Tabletten, geeignet ist.

Es wurde daher nach Lösungsmöglichkeiten gesucht, wie die im Herstellungsprozess in Pulverform anfallende α-Alanin-N,N-diessigsäure in eine Form übergeführt werden kann, die sich zum Einsatz im obigen Prozess der Herstellung von Tabs für Spülmaschinen eignet.

Nach dem Verfahren der EP-A 0 845 456 wird α-Alanin-N,N-diessigsäure-Pulver durch Aufbaugranulation, indem das Pulver mit einer Granulierflüssigkeit gemischt, getrocknet und ausgesiebt wird, in die Form eines Granulates, d.h. eines körnigen und leicht schüttbaren Feststoffes übergeführt, der in kristalliner Form vorliegt und als Trilon^{®} M Granulat der BASF AG bekannt ist. Problematisch ist hierbei jedoch, dass bei der nachfolgenden Weiterverarbeitung des Trilon^{®} M Granulates zu Tabs für Spülmaschinen massive Anbackungen an den Pressstempeln auftreten.

Obwohl die Zusammenhänge nicht vollständig geklärt sind, wird angenommen, dass das Verkleben der Pressstempel durch eine Veränderung des Granulates beim Pressen hervorgerufen wird, wobei das Granulat bricht.

Es ist bekannt, Schüttgüter auf Walzenkompaktoren zu verdichten, beispielsweise auch in der Waschmittelproduktion (vgl. Martin Holl in; Chemie, Anlagen und Verfahren, Ausgabe 08/2001). Hierbei werden die Rohstoffe über einen Fülltrichter, häufig mit Stopfschnecken, einem Walzenspalt zwischen zwei Walzen eines Walzenpaares zugeführt, im Walzenspalt unter Druck verdichtet, unter Erhalt von Schülpen, die in einer Brechanlage in Granulate gewünschter Größe gebrochen und anschließend ausgesiebt werden.

Das α-Alanin-N,N-diessigsäurepulver ist jedoch ohne weitere Zusatzstoffe nicht gut kompaktierbar.

Polyethylenglykole sind als Pluriol^{®} E-Marken der BASF AG bekannt und werden beispielsweise als Konsistenzregler und Bindemittel in Reinigungs- und Spülmitteltabletten, eingesetzt (vgl. Pluriol^{®} E-Marken, technische Information der BASF AG, Mai 2005).

Es war Aufgabe der Erfindung, ein Verfahren zur Herstellung eines rieselfähigen und lagerstabilen Feststoffes enthaltend im Wesentlichen α-Alanin-N,N-diessigsäure und/oder ein oder mehrere Derivate der α-Alanin-N,N-diessigsäure zur Verfügung zu stellen, das zur Herstellung von Tabs für Spülmaschinen durch Vermischen mit weiteren Zusatzstoffen und Tabletten ohne die oben beschriebenen Nachteile einsetzbar ist.

Entsprechend wurde ein Verfahren zur Herstellung eines rieselfähigen und lagerstabilen Feststoffes enthaltend im Wesentlichen α-Alanin-N,N-diessigsäure und/oder ein oder mehrere Derivate der α-Alanin-N,N-diessigsäure gefunden, ausgehend von einem Pulver der α-Alanin-N,N-diessigsäure und/oder einem oder mehreren Derivaten der α-Alanin-N,N-diessigsäure durch
a. Kompaktieren des Pulvers der α-Alanin-N,N-diessigsäure und/oder eines oder mehrerer Derivate der α-Alanin-N,N-diessigsäure auf einer Walzenpresse unter Erhalt von Schülpen,
b. Brechen der Schülpen und
c. Aussieben der gebrochenen Schülpen, das
   dadurch gekennzeichnet ist, dass die Kompaktierung auf der Walzenpresse unter Zusatz von Polyethylenglykol mit einem Schmelzpunkt größer als 35°C durchgeführt wird.

Das Verfahren geht aus von einem Pulver der α-Alanin-N,N-diessigsäure und/oder eines oder mehrerer Derivate der α-Alanin-N,N-diessigsäure, das in einer Walzenpresse unter Druck im Walzenspalt derselben kompaktiert wird. Der Einzug in den Walzenspalt erfolgt üblicherweise über einen Schütttrichter, wobei im Schütttrichter häufig eine Stopfschnecke angeordnet ist.

Aus dem Walzenspalt werden Schülpen erhalten d.h., mehr oder weniger bandförmige Zwischenprodukte, die bei der Walzenkompaktierung durch Verpressen des Ausgangsmaterials entstehen und die anschließend in einer Brechanlage gebrochen und ausgesiebt werden.

Erfindungsgemäß wird der Walzenpresse zusammen mit dem Pulver der α-Alanin-N,N-diessigsäure und/oder eines oder mehrerer Derivate der α-Alanin-N,N-diessigsäure ein Polyethylenglykol mit einem Schmelzpunkt oberhalb von 35°C zugeführt. Die Begrenzung auf Polyethylenglykoltypen mit Schmelzpunkten größer als 35°C ist erforderlich, um sicherzustellen, dass sich das Polyethylenglykol im Kompaktierverfahren nicht verflüssigt.

Bevorzugt werden Polyethylenglykole mit einem Schmelzpunkt größer als 40°C eingesetzt.

Entsprechend werden die unter dem Markennamen Pluriol^{®} der BASF AG bekannten Polyethylenglykoltypen Pluriol^{®} E 1500, Pluriol^{®} E 4000 Pulver oder Pluriol^{®} E 6000-Pulver besonders bevorzugt eingesetzt.

Das Mischungsverhältnis zwischen dem Pulver aus α-Alanin-N,N-diessigsäure und/oder einem oder mehreren Derivaten der α-Alanin-N,N-diessigsäure und dem Polyethylenglykolpulver wird der Gestalt gewählt, dass der Zusatz an Polyethylenglykol zwischen 2 und 15 Gew.-%, bezogen auf das Gesamtgewicht aus α-Alanin-N,N-diessigsäure-Pulver und/oder einem oder mehreren Derivaten der α-Alanin-N,N-diessigsäure und Polyethylenglykol-Pulver, beträgt. Bevorzugt wird der Zusatz an Polyethylenglykol zwischen 5 und 15 Gew.-%, bezogen auf das Gesamtgewicht aus α-Alanin-N,N-diessigsäure-Pulver und/oder einem oder mehreren Derivaten der α-Alanin-N,N-diessigsäure und Polyethylenglykol-Pulver gewählt. Besonders bevorzugt ist ein Zusatz an Polyethylenglykol von 7 Gew.-%, bezogen auf das Gesamtgewicht aus α-Alanin-N,N-diessigsäure-Pulver und/oder einem oder mehreren Derivaten der α-Alanin-N,N-diessigsäure und Polyethylenglykol-Pulver.

Die Kompaktierung in der Walzenpresse wird bevorzugt unter einer Presskraft, bezogen auf die Walzenbreite und den Walzendurchmesser, von 2 bis 10 N/mm x mm, durchgeführt.

Gegenstand der Erfindung ist auch die Verwendung des nach dem oben beschriebenen Verfahren hergestellten rieselfähigen und lagerstabilen Feststoffes zur Herstellung von Tabs für Spülmaschinen durch Vermischen mit weiteren Zusatzstoffen, insbesondere mit Enzymen, Peroxyden und Soda und anschließender Tablettierung.

Es wurde somit ein Verfahren gefunden, das die Kompaktierbarkeit von Pulvern aus α-Alanin-N,N-diessigsäure und/oder einem oder mehreren Derivaten derselben verbessert und gleichzeitig deren Hygroskopisität erniedrigt, unter Einsatz eines für die bestimmungsgemäße Anwendung, zur Herstellung von Tabs für Spülmaschinen, verfahrenseigenen Hilfsstoffes.

Die Erfindung wird im Folgenden anhand einer Figur sowie von Ausführungsbeispielen näher erläutert.

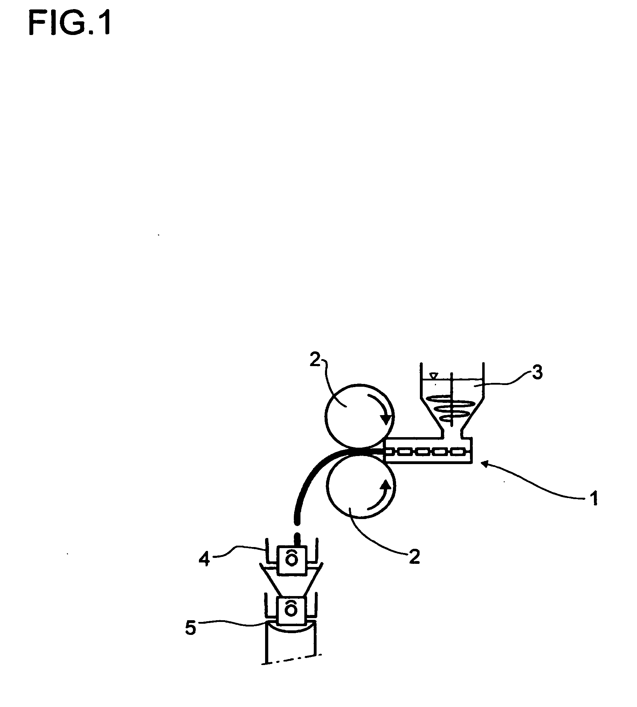

Die einzige Figur 1 zeigt die schematische Darstellung einer Walzenpresse 1 mit zwei gegensinnig rotierenden Walzen 2, mit Zuführung des zu kompaktierenden Pulvers in der in der Figur dargestellten beispielhaften Ausführungsform über einen Schütttrichter 3, unter Bildung von Schülpen, die in einer Brechanlage 4 zu gewünschter Größe gebrochen und in einer Siebanlage 5 ausgesiebt werden.

### Ausführungsbeispiele:

In einem Laborkompaktor^{®} L200/50 der Firma Hosokawa Bepex wurde Trilon^{®} MX der BASF AG (α-Alanin-N,N-diessigsäure) unter Erhalt von Schülpen kompaktiert. Die Schülpen wurden gebrochen und anschließend ausgesiebt.

Zusätzlich zu Trilon^{®} MX wurden Polyethylenglykole mit unterschiedlichem Polymerisationsgrad und entsprechend mit unterschiedlichem Schmelzpunkt eingesetzt, und zwar Polyethylenglykoltypen der Marke Pluriol^{®} der BASF AG mit einer mittleren molaren Masse von 4000 (Pluriol^{®} E 4000) bzw. mit einer mittleren molaren Masse von 6000 (Pluriol^{®} E 6000).

Der Laborkompaktor^{®} L 200/50 war mit Walzen mit 6 mm Feinprofil bestückt, die mit einer Drehzahl von 7,5 Umdrehungen pro Minute betrieben wurden. Die Presskraft an den Walzen, bezogen auf die Walzenbreite und den Walzendurchmesser, betrug ca. 8 bis 10 N/mm x mm.

Die erhaltenen Schülpen wurden gebrochen und in einer Siebanlage auf drei Siebfraktionen, einer ersten Fraktion mit einer Siebgröße von 0,25 mm, einer mittleren Fraktion mit einer Siebgröße von 0,355 bis 1,25 mm, und einer dritten Fraktion mit einer Siebgröße von kleiner als 0,355 mm, gesiebt.

### Vergleichsversuche:

Trilon^{®} MX wurde zum Vergleich ohne Zusatz von Polyethylenglykol kompaktiert: hierbei wurde ein Produkt erhalten, das im nachfolgenden Verfahren der Verpressung zu Tabletten für die Herstellung von Tabs für Spülmittel zum Verkleben der Pressstempel führte.

In einem weiteren Vergleichsversuch wurde Trilon^{®} MX zusammen mit 20 Gew.-% Pluriol^{®} E 4000 der BASF AG kompaktiert: dabei stieg die Temperatur des Produktes beim Kompaktieren stark an, so dass eine Zerkleinerung desselben durch Mahlen erst nach Kühlung möglich war.

In einem weiteren Vergleichversuch wurde ein Polyethylenglykol mit einer höheren mittleren molaren Masse von 6000, und zwar Pluriol^{®} E 6000 der BASF AG, eingesetzt. Es wurde ein Produkt erhalten, das zwar verfahrenstechnisch für den Einsatz in einem nachfolgenden Verfahren zur Herstellung von Tabs für Spülmaschinen geeignet, jedoch in der Endanwendung auf Grund der hohen Molekularmasse ungeeignet ist.

In einem erfindungsgemäßen Ausführungsbeispiel wurde Trilon^{®} MX zusammen mit 7 Gew.% Pluriol^{®} E 4000 der BASF AG, bezogen auf das Gesamtgewicht aus Trilon^{®} MX und Pluriol^{®} E 4000, auf dem oben beschriebenen Laborkompaktor^{®} L 200/50 kompaktiert. Nach Brechen der Schülpen und Sieben auf die drei oben angegebenen Siebfraktionen wurde ein Granulat mit den folgenden Siebfraktionen erhalten: Siebfraktion mit einer Siebgröße von 1,25 mm 20,81 %, Siebfraktion mit einer Siebgröße von zwischen 0,355 mm und 1,25 mm 38,81 % und Siebfraktion mit einer Siebgröße von kleiner als 0,355 mm 40,38%.

Das erhaltene Granulat konnte problemlos, nach Vermischen mit Zusatzstoffen, zu Tabs für Spülmaschinen verpresst werden.

## Patentansprüche

1. Verfahren zur Herstellung eines rieselfähigen und lagerstabilen Feststoffes enthaltend im Wesentlichen α-Alanin-N,N-diessigsäure und/oder ein oder mehrere Derivate der α-Alanin-N,N-diessigsäure, ausgehend von einem Pulver der α-Alanin-N,N-diessigsäure und/oder einem oder mehreren Derivaten der α-Alanin-N,N-diessigsäure durch
a. Kompaktieren des Pulvers der α-Alanin-N,N-diessigsäure und/oder einem oder mehreren Derivaten der α-Alanin-N,N-diessigsäure auf einer Walzenpresse unter Erhalt von Schülpen,
b. Brechen der Schülpen und
c. Aussieben der gebrochenen Schülpen,
**dadurch gekennzeichnet, dass** die Kompaktierung auf der Walzenpresse unter Zusatz von Polyethylenglykol mit einem Schmelzpunkt größer als 35°C durchgeführt wird und wobei der Zusatz an Polyethylenglykol zur Kompaktierung auf der Walzenpresse 2 bis 15 Gew.-%, bezogen auf das Gesamtgewicht des der Kompaktierung auf der Walzenpresse zugeführten Pulvers aus α-Alanin-N,N-diessigsäure und/oder einem oder mehreren Derivaten der α-Alanin-N,N-diessigsäure und Polyethylenglykol, beträgt.

2. Verfahren nach Anspruch 1, **dadurch gekennzeichnet, dass** die Kompaktierung auf der Walzenpresse unter Zusatz von Polyethylenglykol mit einem Schmelzpunkt größer als 40°C durchgeführt wird.

3. Verfahren nach Anspruch 1 oder 2, **dadurch gekennzeichnet, dass** der Zusatz an Polyethylenglykol zur Kompaktierung auf der Walzenpresse 5 bis 15 Gew.-%, bezogen auf das Gesamtgewicht des der Kompaktierung auf der Walzenpresse zugeführten Pulvers aus α-Alanin-N,N-diessigsäure und/oder einem oder mehreren Derivaten der α-Alanin-N,N-diessigsäure und Polyethylenglykol, beträgt.

4. Verfahren nach Anspruch 3, **dadurch gekennzeichnet, dass** der Zusatz an Polyethylenglykol zur Kompaktierung auf der Walzenpresse 7 Gew.-%, bezogen auf das Gesamtgewicht des der Kompaktierung auf der Walzenpresse zugeführten Pulvers aus α-Alanin-N,N-diessigsäure und/oder einem oder mehreren Derivaten der α-Alanin-N,N-diessigsäure und Polyethylenglykol, beträgt.

5. Verfahren nach einem der Ansprüche 1 bis 4, **dadurch gekennzeichnet, dass** die Kompaktierung in der Walzenpresse mit einer Presskraft an den Walzen bezogen auf die Walzenbreite und den Walzendurchmesser von 2 bis 10 N/mm x mm durchgeführt wird.

6. Verwendung des in einem Verfahren nach einem der Ansprüche 1 bis 5 hergestellten rieselfähigen und lagerstabilen Feststoffes enthaltend im Wesentlichen α-Alanin-N,N-diessigsäure und/oder ein oder mehrere Derivaten der α-Alanin-N,N-diessigsäure zur Herstellung von Tabs für Spülmaschinen durch Vermischen mit weiteren Zusatzstoffen und Verpressen zu Tabs.

## Claims

1. A process for producing a free-flowing and storage-stable solid comprising essentially a-alanine-N,N-diacetic acid and/or one or more derivatives of α-alanine-N,N-diacetic acid proceeding from a powder of α-alanine-N,N-diacetic acid and/or one or more derivatives of α-alanine-N,N-diacetic acid by
a. compacting the powder of α-alanine-N,N-diacetic acid and/or one or more derivatives of α-alanine-N,N-diacetic acid on a roller press to obtain slugs,
b. crushing the slugs and
c. screening the crushed slugs,
which comprises performing the compaction on the roller press with addition of polyethylene glycol with a melting point greater than 35°C and the addition of polyethylene glycol for compaction on the roller press being from 2 to 15% by weight, based on the total weight of the powder composed of α-alanine-N,N-diacetic acid and/or one or more derivatives of α-alanine-N,N-diacetic acid and polyethylene glycol supplied to the compaction on the roller press.

2. The process according to claim 1, wherein the compaction on the roller press is carried out with addition of polyethylene glycol with a melting point greater than 40°C.

3. The process according to claim 1 or 2, wherein the addition of polyethylene glycol for compaction on the roller press is from 5 to 15% by weight, based on the total weight of the powder composed of α-alanine-N,N-diacetic acid and/or one or more derivatives of α-alanine-N,N-diacetic acid and polyethylene glycol supplied to the compaction on the roller press.

4. The process according to claim 3, wherein the addition of polyethylene glycol for compaction on the roller press is 7% by weight, based on the total weight of the powder composed of α-alanine-N,N-diacetic acid and/or one or more derivatives of α-alanine-N,N-diacetic acid and polyethylene glycol supplied to the compaction on the roller press.

5. The process according to any of claims 1 to 4, wherein the compaction in the roller press is carried out with a pressure on the rollers, based on the roller width and the roller diameter, of from 2 to 10 N/mm x mm.

6. The use of the free-flowing and storage-stable solid comprising essentially α-alanine-N,N-diacetic acid and/or one or more derivatives of α-alanine-N,N-diacetic acid produced in a process according to any of claims 1 to 5 for producing tabs for machine dishwashers by mixing with further additives and pressing to tabs.

## Revendications

1. Procédé pour la production d'un solide apte à l'écoulement et stable au stockage, contenant essentiellement de l'acide α-alanine-N,N-diacétique et/ou un ou plusieurs dérivés de l'acide α-alanine-N,N-diacétique à partir d'une poudre de l'acide α-alanine-N,N-diacétique et/ou d'un ou plusieurs dérivés de l'acide α-alanine-N,N-diacétique, par
a. compactage de la poudre de l'acide α-alanine-N,N-diacétique et/ou d'un ou plusieurs dérivés de l'acide α-alanine-N,N-diacétique, sur une presse à cylindres, avec obtention de corps intermédiaires plats,
b. concassage des corps, intermédiaires plats,
c. tamisage des corps intermédiaires plats concassés,
**caractérisé en ce que** le compactage est effectué sur la presse à cylindres avec addition de polyéthylèneglycol ayant un point de fusion supérieur à 35 °C et l'addition de polyéthylèneglycol dans le compactage sur la presse à cylindres étant de 2 à 15 % en poids, par rapport au poids total de la poudre d'acide α-alanine-N,N-diacétique et/ou d'un ou plusieurs dérivés de l'acide α-alanine-N,N-diacétique, envoyée au compactage sur la presse à cylindres, et du polyéthylèneglycol.

2. Procédé selon la revendication 1, **caractérisé en ce qu'**on effectue le compactage sur la presse à cylindres avec addition de polyéthylèneglycol ayant un point de fusion supérieur à 40 °C.

3. Procédé selon la revendication 1 ou 2, **caractérisé en ce que** dans le compactage sur la presse à cylindres l'addition de polyéthylèneglycol est de 5 à 15 % en poids, par rapport au poids total de la poudre d'acide α-alanine-N,N-diacétique et/ou d'un ou plusieurs dérivés de l'acide α-alanine-N,N-diacétique, envoyée au compactage sur la presse à cylindres, et du polyéthylèneglycol.

4. Procédé selon la revendication 3, **caractérisé en ce que** dans le compactage sur la presse à cylindres l'addition de polyéthylèneglycol est de 7 % en poids, par rapport au poids total de la poudre d'acide α-alanine-N,N-diacétique et/ou d'un ou plusieurs dérivés de l'acide α-alanine-N,N-diacétique, envoyée au compactage sur la presse à cylindres, et du polyéthylèneglycol.

5. Procédé selon l'une quelconque des revendications 1 à 4, **caractérisé en ce que** le compactage dans la presse à cylindres est effectué avec une force de compression sur les cylindres, par rapport à la largeur des cylindres et au diamètre des cylindres, de 2 à 10 N/mm × mm.

6. Utilisation du solide apte à l'écoulement et stable au stockage, contenant essentiellement de l'acide α-alanine-N,N-diacétique et/ou un ou plusieurs dérivés de l'acide α-alanine-N,N-diacétique, produit selon l'une quelconque des revendications 1 à 5, pour la fabrication de pastilles pour lave-vaisselle, par mélange avec d'autres additifs et pressage en pastilles.
